# EUROPÄISCHE PATENTANMELDUNG

(11) **EP 1 977 728 A2**
(43) Veröffentlichungstag der Anmeldung: **08.10.2008**
(21) Anmeldenummer: 08001460.8
(22) Anmeldetag: 26.01.2008
(51) Int. Cl.: A61K 8/11, A61K 8/27, A61K 8/39, A61Q 5/12

(54) **Stabilisierung tensidhaltiger Mittel**

(30) Priorität: 04.04.2007 DE 102007016708
(71) Anmelder: Henkel AG & Co. KGaA, 40589 Düsseldorf (DE)
(72) Erfinder: Hippe, Thomas, 25482 Appen (DE); Schulze Zur Wieschke, Erik, 20251 Hamburg (DE); Scheunemann, Volker, 21339 Lüneburg (DE); Schröder, Thomas, 22395 Hamburg (DE)

(57) **Zusammenfassung**

Es werden tensidhaltige Mittel zur Behandlung keratinischer Fasern beschrieben, die zur Stabilisierung darin unlöslicher Metalloxid-Partikel gehärtetes Rizinusöl enthalten.

Durch die Stabilisierung der Metalloxidpartikel mit gehärtetem Rizinusöl in einer Tensidbasis ist es möglich eine Vielzahl kosmetischer Präparate herzustellen, in denen das Metalloxid auch nach einem längeren Zeitraum stabil dispergiert vorliegt und seine antiseptische Wirksamkeit entfalten kann.

## Beschreibung

Die Erfindung betrifft tensidhaltige Mittel zur Behandlung keratinischer Fasern, die zur Stabilisierung eines darin unlöslichen Metalloxids gehärtetes Rizinusöl umfassen, ein kosmetisches Verfahren zur Stabilisierung unlöslicher Metalloxide in tensidhaltigen Formulierungen sowie die Verwendung von gehärtetem Rizinusöl zur Stabilisierung unlöslicher Metalloxidpartikel in tensidischen Formulierungen.

Bedingt durch das stetig steigende Hygiene-Empfinden der Menschen und die damit immer größer und breiter werdenden Anforderungen an kosmetische Produkte ergeben sich fortwährend neue Problemstellungen und Schwierigkeiten bei deren Herstellung.

Um den Bedürfnissen der Verbraucher gerecht zu werden, müssen Reinigungsmittel für Haut und Haar, wie sie beispielsweise als flüssige Seifen, Shampoos, Duschbäder, Schaumbäder, Dusch- und Waschgele im Handel erhältlich sind, heutzutage nicht nur ein gutes Reinigungsvermögen aufweisen, sondern sollen weiterhin für die Haut und das Haar gut verträglich sein und auch bei häufiger Anwendung nicht zu starker Entfettung oder Trockenheit führen.

Diesen Ansprüchen kann durch die gezielte Wahl geeigneter Wirkstoffe entsprochen werden, doch gerade die Kombination einer Vielzahl unterschiedlicher Wirkstoffe kann unerwünschte Wechselwirkungen unter der Wirkstoffen hervorrufen, oder es kann eine unerwünschte pH-Wert-Verschiebung stattfinden, wodurch die Wirksamkeit einiger Komponenten nachlässt oder gar verloren geht. Des weiteren existiert eine Fülle wertvoller kosmetischer Wirkstoffe, die in einem üblichen kosmetischen Medium unlöslich sind und mithilfe geeigneter Techniken und Hilfsmittel über einen langen Zeitraum stabil emulgiert, suspendiert, dispergiert oder in eine Aerosolform gebracht werden müssen.

Die Herstellung kosmetischer Produkte mit neuartigen Wirkungen ist daher ein sensibles Thema und stellt den Fachmann bei jeder Veränderung vor mannigfaltige formulierungstechnische Schwierigkeiten.

So ist die Konservierung kosmetischer Mittel auf Tensidbasis ein dauerhaftes Problem bei der Entwicklung neuer Produkte, denn nahezu alle kosmetischen Haut- und Haarreinigungsmittel beruhen auf einer wässrigen Tensidbasis, die eine sehr bevorzugte Basis für unerwünschte Mikroorganismen bildet.
Diese gilt es zu bekämpfen, um durch Mikroorganismen bedingte Veränderungen der kosmetischen Präparate wie Geruch, Aussehen, Konsistenz, Gärung oder Schimmelbildung zu vermeiden.

Nun müssen antiseptische Mittel, die für den Einsatz in kosmetischen Präparaten bestimmt sind, in erster Linie gut hautverträglich sein. Gleichzeitig müssen sie sich aber auch in die gewünschten Produkte gut einarbeiten und darin stabilisieren lassen und sie müssen in einem pH-Bereich, der für das jeweilige kosmetische Mittel üblich ist, wirksam sein.

Eine neuartige Wirkstoffgruppe mit hervorragenden antiseptischen Eigenschaften stellen modifizierte Metalloxide dar. Es konnte beobachtet werden, dass ihre antiseptische Wirkung besonders stark ist, wenn die Metalloxide in der Form besonders kleiner Teilchen, insbesondere in der Form nanopartikulärer Teilchen, ungelöst, aber stabil dispergiert in der Tensidbasis vorliegen.

Problematisch bei der Herstellung geeigneter Zusammensetzungen, enthaltend nanopartikuläre ungelöste Metalloxidpartikel, war folglich, ein Stabilisierungsmittel für die Metalloxidpartikel zu finden, das eine dauerhafte Dispergierung der Metalloxidpartikel gewährleistet. Gleichzeitig musste ein geeigneter pH-Bereich gefunden werden, um eine optimale Wirksamkeit der Metalloxidpartikel bei optimaler Stabilisierung durch das Stabilisierungsmittel zu gewährleisten.
Im Idealfall sollte es sogar möglich sein, mindestens ein weiteres Konservierungsmittel in die Tensidbasis einzuarbeiten, das zusammen mit dem Metalloxid in einer lagerstabilen Formulierung einen synergistischen Effekt aufweist.

Überraschenderweise wurden diese Probleme gelöst, indem hydriertes Rizinusöl als geeignetes Stabilisierungsmittel für in einer Tensidbasis unlösliche, nanopartikuläre Metalloxidpartikel gefunden wurde.

Durch die Kombination dieser beiden Stoffe konnten eine Vielzahl kosmetischer Präparate hergestellt werden, in denen das Metalloxid auch nach einem längeren Zeitraum stabil dispergiert vorlag und seine Wirksamkeit entfalten konnte, wodurch eine lange Lagerstabilität der Präparate ohne das Auftreten der oben genannten Nachteile erzielt werden konnte.

Gegenstand der Erfindung sind damit tensidhaltige Mittel zur Behandlung keratinischer Fasern, die zur Stabilisierung darin unlöslicher Metalloxid-Partikel gehärtetes Rizinusöl enthalten.

Bevorzugte erfindungsgemäße Mittel enthalten ein- oder mehrfach beschichtete, nanopartikuläre Metalloxidpartikel, die in den Mitteln - bezogen auf das Gesamtgewicht des Mittels - in einer Menge von 0,05 bis 10,0 Gew.-%, bevorzugt in einer Menge von 0,1 bis 5 Gew.-% und insbesondere in einer Menge von 0,5 bis 2,0 Gew.-% eingesetzt werden.

Als Beschichtungsmaterial für die Metalloxidpartikel eignen sich erfindungsgemäß Alkylethercarbonsäuren und Polyetheralkylether und/oder Polyethylenglycolalkylether.

In einer besonders bevorzugten Ausführungsform der Erfindung sind die Metalloxidpartikel zusammengesetzt aus
- 40 bis 90 Gew.-% nanopartikulärem Metalloxid,
- 1 bis 30 Gew.-% Alkylethercarbonsäure(n) und
- 1 bis 30 Gew.-% Polyetheralkylether(n) oder Polyethylenglycolalkylethern und insbesondere aus
- 60 bis 75 Gew.-% nanopartikulärem Metalloxid,
- 5 bis 25 Gew.-% Alkylethercarbonsäure(n) und
- 5 bis 25 Gew.-% Polyetheralkylether(n) oder Polyethylenglycolalkylethern .

Erfindungsgemäß geeignete Alkylethercarbonsäuren weisen eine Alkylkettenlänge von 6 bis 22 C-Atomen, bevorzugt von 8 bis 18 C-Atomen und insbesondere von 10 bis 14 C-Atomen auf.

Erfindungsgemäß geeignete Polyetheralkylether weisen einen Ethoxylierungsgrad von 0 bis 20, insbesondere von 8 bis 12, und eine Alkylkettenlänge von 4 bis 22 C-Atomen, bevorzugt von 6 bis 18 C-Atomen und insbesondere von 8 bis 16 C-Atomen auf.

Bei den erfindungsgemäß geeigneten Metalloxidpartikeln handelt es sich üblicherweise um Zinkoxid- und/oder Titanoxidpartikel. Erfindungsgemäß besonders geeignet sind ein- oder mehrfach beschichtete, nanopartikuläre Zinkoxidpartikel.

Die erfindungsgemäße Wirkstoffkombination entfaltet seinen antiseptischen Effekt besonders vorteilhaft in einem pH-Bereich von 4,0 bis 9,0 und insbesondere in einem pH-Bereich von 7,0 bis 8,0.

Erfindungsgemäß bevorzugte Mittel sind Reinigungsmittel für die Haut und die Haare, die ein Gemisch aus mindestens einem anionischen Tensid mit einem amphoteren und/oder zwitterionischen und/oder einem nichtionischen Tensid umfassen.

Der Gesamttensidgehalt in den Reinigungsmitteln beträgt 5 bis 35%, bevorzugt 7 bis 25% und insbesondere 8 bis 15% - bezogen auf das Gesamtgewicht des Reinigungsmittels.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 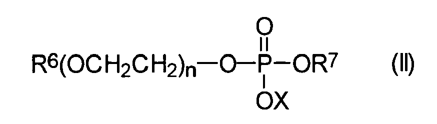 in der R⁶ bevorzugt für einen aliphatischen Kohlenwasserstoffrest mit 8 bis 30 Kohlenstoffatomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁ bis C₄ - Kohlenwasserstoffrest, steht,
- sulfatierte Fettsäurealkylenglykolester der Formel (III),

   R¹²CO(AlkO)ₙSO₃M (III)

   in der R¹²CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV), 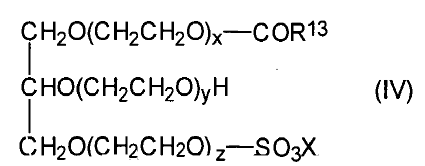 in der R¹³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (VIII) eingesetzt, in der R¹³CO für einen linearen Acylrest mit 8 bis 18 Kohlenstoffatomen steht.

Bevorzugte anionische Tenside sind Alkylsulfate, Alkylpolyglykolethersulfate und Ethercarbonsäuresalze mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe, Sulfobernsteinsäuremono-alkylpolyoxyethylester mit 8 bis 18 C-Atomen in der Alkylgruppe und 1 bis 6 Oxyethylgruppen sowie die Alkali- und/oder Ammoniumsalze des Tridecethsulfats.

Besonders bevorzugte anionische Tenside sind die Alkali- oder Ammoniumsalze des Laurylethersulfates mit einem Ethoxylierungsgrad von 2 bis 4 EO sowie die Alkali- und/oder Ammoniumsalze des Tridecethsulfats.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈- C₂₄- Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 von der Firma SHC im Handel erhältlich ist,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (V)

   R¹⁴CO-(OCH₂CHR¹⁵)_{w}OR¹⁶ (V)

   in der R¹⁴CO für einen linearen oder verzweigten, gesättigten und/oder ungesättigten Acylrest mit 6 bis 22 Kohlenstoffatomen, R¹⁵ für Wasserstoff oder Methyl, R¹⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

In einigen erfindungsgemäß bevorzugten Ausführungsformen haben sich Tensidgemische aus vernetzten oder unvernetzten anionischen Tensiden wie beispielsweise Natriumtridecethsulfat, amphoteren Tensiden wie beispielsweise Natrium Lauroamphoacetat und nichtionischen Tensiden wie beispielsweise Cocamide MEA, wie sie beispielsweise unter der Handelsbezeichnung Miracare SLB 365 MP im Handel erhältlich sind, als besonders geeignete Tensidbasis herausgestellt.

Gemäß einer weiteren Ausführungsform der Erfindung können die tensidhaltigen Reinigungsmittel weiterhin kationische Tenside vom Typ der quartären Ammoniumverbindungen, der Esterquats und der Amidoamine enthalten.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®}C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei dem erfindungsgemäß zwingend vorhandenen Rizinusöl handelt es sich beispielsweise um das unter der INCI-Bezeichnung "Hydrogenated Castor Oil" bekannte hydrierte Rizinusöl, wie es beispielsweise von der Firma Cognis unter der Bezeichnung Cutina HR^{®} im Handel erhältlich ist.

Das Hydrogenated Castor Oil wird in den erfindungsgemäßen Zusammensetzungen - bezogen auf ihr Gesamtgewicht - in einer Menge von 0,01 bis 5% bevorzugt in einer Menge von 0,05 bis 3% und insbesondere in einer Menge von 0,1 bis 2%, eingesetzt.

In einer weiteren bevorzugten Ausführungsform der Erfindung enthalten die erfindungsgemäßen Mittel mindestens ein weiteres Konservierungsmittel, ausgewählt aus der Gruppe, die gebildet wird aus Benzoesäure, Propionsäure, Salicylsäure und/oder Sorbinsäure, sowie aus den kosmetisch verträglichen Salzen und Estern dieser Säuren, aus Formaldehyl und/oder Paraformaldehyd, aus Benzylalkohol, Germall, Dowicil, Kathon, Baypival, Methylisothiazoline, quaternären Ammoniumverbindungen, Alkoholen und/oder aus etherischen Ölen wie Teebaumöl, Cineol, Eugenol, Geraniol, Limonen, Menthol und/oder Thymol.

Erfindungsgemäß ganz besonders bevorzugte weitere Konservierungsmittel sind Benzoesäure, Salicylsäure und/oder ein kosmetisch verträgliches Salz oder ein kosmetisch verträglicher Ester dieser Säuren, Benzylalkohol, Dowicil, Kathon, Baypival, Methylisothiazoline sowie Gemische dieser Stoffe. Insbesondere bevorzugt ist Methylisothiazoline oder einem Gemisch aus Methylisothiazoline mit einem der oben genannten Stoffe.

Für den Fall, in dem das erfindungsgemäße Mittel als Shampoo konfektioniert wird, kann es bevorzugt sein, dass dieses mindestens ein Antischuppenmittel in einer Menge von 0,05 bis 5 Gew.-%, bevorzugt von 0,1 bis 3,0 Gew.-% und insbesondere von 0,3 bis 2,0 Gew.-% enthält (bezogen auf das gesamte Mittel).

Diese(s) ist (sind) beispielsweise ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

In einer weiteren bevorzugten Ausführungsform der Erfindung enthält das erfindungsgemäße Mittel weiterhin mindestens einen Vertreter aus der Gruppe der kationischen Polymere, der wasserunlöslichen Wachs- und Ölkomponenten, der Vitamine, der Provitamine, der Proteinhydrolysate, der Pflanzenextrakte, der UV-Filter, der Aminosäuren, der wasserunlöslichen Silikone, der wasserlöslichen Silikone und/oder der Amodimethicone.

Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (VI), 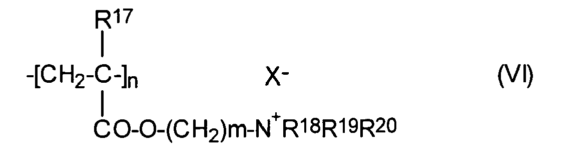 in der R¹⁷ = -H oder -CH₃ ist, R¹⁸, R¹⁹ und R²⁰ unabhängig voneinander ausgewählt sind aus C₁₋₄-Alkyl-, -Alkenyl- oder-Hydroxyalkylgruppen, m = 1, 2, 3 oder 4, n eine natürliche Zahl und X-ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2.

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

In einer besonders bevorzugten Ausführungsform der Erfindung ist als kationisches Polymer mindestens ein Polymer aus der Gruppe der kationischen Guar-Derivate und/oder Polyquaternium-7 (Merquat 550), Polyquaternium-6, Polyquaternium-10 und/oder Polyquaternium-67 (SoftCat^{®}-Polymere) in den Reinigungsmitteln enthalten.

Das oder die kationische(n) Polymer(e) ist (sind) in den erfindungsgemäßen Mitteln - bezogen auf das gesamte Mittel - in Mengen von 0,1 bis 5 Gew.-% enthalten. Mengen von 0,2 bis 3, insbesondere von 0,5 bis 2 Gew.-%, sind besonders bevorzugt.

In einer weiteren bevorzugten Ausführungsform der Erfindung enthalten die tensidhaltigen Reinigungsmittel außerdem mindestens einen Bestandteil aus der Gruppe der wasserunlöslichen Ölkomponenten, der Vitamine, der Provitamine, der Proteinhydrolysate, der Pflanzenextrakte, der UV-Filter, der Aminosäuren, der wasserunlöslichen Silikone, der wasserlöslichen Silikone und/oder der Amodimethicone.

Als wasserunlösliche Ölkomponenten eignen sich erfindungsgemäß pflanzliche, mineralische oder synthetische Öle, sowie Gemische dieser Komponenten.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Aprikosenkernöl, Babssuöl, Olivenöl, Weizenkeimöl, Kürbiskernöl, Malvenöl, Haselnussöl, Safloröl, Canolaöl, Sasanquaöl, Jojobaöl und Shea-Butter.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen, insbesondere Dialkyl- und Alkylarylsilikone, wie beispielsweise Dimethylpolysiloxan und Methylphenylpolysiloxan, sowie deren hydroxyterminierte, alkoxylierte und quaternierte Analoga in Betracht. Beispiele für solche Silikone sind die von Dow Corning unter den Bezeichnungen DC 190, DC 200, DC 344 und DC 345 (Cyclomethicone) vertriebenen Produkte.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Die erfindungsgemäßen Mittel enthalten die wasserunlösliche Ölkomponente bevorzugt in einem Mengenbereich von 0,1 bis 5 Gew.-%, insbesondere von 0,5 bis 3 Gew.-%, bezogen auf das Gesamtgewicht des Mittels.

In einer weiteren bevorzugten Ausführungsform der Erfindung kann die Wirkung der erfindungsgemäßen Wirkstoffkombination durch weitere Fettstoffe noch weiter optimiert werden. Unter weiteren Fettstoffen sind zu verstehen Fettsäuren, Fettalkohole sowie natürliche und synthetische Wachse, welche sowohl in fester Form als auch flüssig in wäßriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), lsononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinatl-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12, Cetiol^{®} HE oder Cutina^{®} MD.

Die Einsatzmenge der weiteren Fettstoffe beträgt 0,1 - 20 Gew.% bezogen auf das gesamte Mittel. Bevorzugt sind 0,1 - 10 Gew.% und besonders bevorzugt 0,1 - 5 Gew.%, bezogen auf das gesamte Mittel.

Die Gesamtmenge an Öl- und Fettkomponenten in den erfindungsgemäßen Mitteln beträgt üblicherweise 0,01 - 15 Gew.-%, bezogen auf das gesamte Mittel. Mengen von 0,05- 5 Gew.-% sind erfindungsgemäß bevorzugt.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,01-1 Gew.-%, bezogen auf die gesamte Zubereitung.

Zur Vitamin B-Gruppe oder zu dem Vitamin B-Komplex gehören u. a.
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate.
Vitamin B₆ (Pyridoxin sowie Pyridoxamin und Pyridoxal).
   Die genannten Verbindungen der Vitamin B-Gruppe sind in den erfindungsgemäßen Mitteln bevorzugt in Mengen von 0,01 - 2 Gew.-%, bezogen auf das gesamte Mittel, enthalten. Mengen von 0,03 - 1 Gew.-% sind besonders bevorzugt.

Vitamin C (Ascorbinsäure). Vitamin C wird in den erfindungsgemäßen Mitteln bevorzugt in Mengen von 0,01 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,01-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zubereitungen Vitamine, Provitamine und Vitaminvorstufen aus den Gruppen A, E, F und H. Selbstverständlich können auch mehrere Vitamine und Vitaminvorstufen gleichzeitig enthalten sein.

Die Gesamt-Einsatzmenge der Vitamine, Provitamine, Vitaminvorstufen sowie deren Derivate in den erfindungsgemäßen Mitteln beträgt - bezogen auf das Gesamtgewicht des Mittels - 0,01 bis 5 Gew.-%, vorzugsweise 0,02 bis 4 Gew.-% und insbesondere 0,05 bis 3 Gew.-%.

Als Proteinhydrolysate im Sinne der Erfindung werden Proteinhydrolysate und/oder Aminosäuren und deren Derivate (H) verstanden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Die Proteinhydrolysate oder deren Derivate sind in den erfindungsgemäßen Zubereitungen bevorzugt in Mengen von 0,1 bis 10 Gew.-%, bezogen auf das gesamte Mittel, enthalten. Mengen von 0,1 bis 5 Gew.-% sind besonders bevorzugt.

Erfindungsgemäß bevorzugt sind tensidhaltige Reinigungsmittel, die Pflanzenextrakte aus grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Vanille, Eibisch, Meristem, Ginseng und Ingwerwurzel enthalten.

Besonders bevorzugt sind erfindungsgemäß die Extrakte aus Grünem Tee, Mandel, Aloe Vera, Kokosnuß, Mango, Aprikose, Limone, Weizen, Vanille, Kiwi und Melone und insbesondere bevorzugt die Extrakte aus Aloe Vera, Vanille und Melone.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Die Pflanzenextrakte werden in den erfindungsgemäßen Mitteln - bezogen auf deren Gewicht - in einer Menge von 0,01 bis 5 Gew.-%, vorzugsweise 0,02 bis 4 Gew.-% und insbesondere 0,05 bis 3 Gew.-% eingesetzt.

Weiterhin kann in einer bevorzugten Ausführungsform der Erfindung die Wirkung der Zubereitungen durch UV - Filter gesteigert werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul^{®}M 40, Uvasorb^{®}MET, Neo Heliopan^{®}BB, Eusolex^{®}4360), 2-Phenylbenzimidazol-5-sulfonsäure und deren Kalium-, Natrium- und Triethanolaminsalze (Phenylbenzimidazole sulfonic acid; Parsol^{®}HS; Neo Heliopan^{®}Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol^{®}1789, Eusolex^{®}9020), α-(2-Oxoborn-3-yliden)-toluol-4-sulfonsäure und deren Salze, ethoxylierte 4-Aminobenzoesäure-ethylester (PEG-25 PABA; Uvinul^{®}P 25), 4-Dimethylaminobenzoesäure-2-ethylhexylester (Octyl Dimethyl PABA; Uvasorb^{®}DMO, Escalol^{®}507, Eusolex^{®}6007), Salicylsäure-2-ethylhexylester (Octyl Salicylat; Escalol^{®}587, Neo Heliopan^{®}OS, Uvinul^{®}O18), 4-Methoxyzimtsäure-isopentylester (Isoamyl p-Methoxycinnamate; Neo Heliopan^{®}E 1000), 4-Methoxyzimtsäure-2-ethylhexyl-ester (Octyl Methoxycinnamate; Parsol^{®}MCX, Escalol^{®}557, Neo Heliopan^{®}AV), 2-Hydroxy-4-methoxybenzophenon-5-sulfonsäure und deren Natriumsalz (Benzophenone-4; Uvinul^{®}MS 40; Uvasorb^{®}S 5), 3-(4'-Methylbenzyliden)-D,L-Campher (4-Methylbenzylidene camphor; Parsol^{®}5000, Eusolex^{®}6300), 3-Benzyliden-campher (3-Benzylidene camphor), 4-Isopropylbenzylsalicylat, 2,4,6-Trianilino-(p-carbo-2'-ethylhexyl-1'-oxi)-1,3,5-triazin, 3-lmidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb^{®}20 H, Uvinul^{®}400), 1,1'-Diphenylacrylonitrilsäure-2-ethylhexyl-ester (Octocrylene; Eusolex^{®}OCR, Neo Heliopan^{®}Type 303, Uvinul^{®}N 539 SG), o-Aminobenzoesäure-menthylester (Menthyl Anthranilate; Neo Heliopan^{®}MA), 2,2',4,4'-Tetrahydroxybenzophenon (Benzophenone-2; Uvinul^{®}D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, α-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der Regel ein Wasserstoffatom, des UV-Filters durch eine kationische Gruppe Q, insbesondere mit einer quartären Aminofunktion, ersetzt wird.
Verbindungen, von denen sich der Strukturteil U ableiten kann, sind beispielsweise
- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.

Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.

Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.

Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.

Vorteilhafterweise hat die Gruppe Q die allgemeine Struktur -(CH₂)ₓ-N⁺R¹R²R³ X⁻, in der x steht für eine ganze Zahl von 1 bis 4, R¹ und R² unabhängig voneinander stehen für C₁₋₄-Alkylgruppen, R³ steht für eine C₁₋₂₂-Alkylgruppe oder eine Benzylgruppe und X⁻ für ein physiologisch verträgliches Anion. Im Rahmen dieser allgemeinen Struktur steht x bevorzugt für die die Zahl 3, R¹ und R² jeweils für eine Methylgruppe und R³ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.

Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Gemäß einer weiteren bevorzugten Ausführungsform der Erfindung enthalten die Mittel außerdem mindestens ein flüchtiges oder nichtflüchtiges, wasserunlösliches Silikon, wasserlösliches und/oder aminofuktionalisiertes Silikon.

Erfindungsgemäß geeignete Silikone bewirken die unterschiedlichsten Effekte. So beeinflussen sie beispielsweise gleichzeitig die Trocken- und Naßkämmbarkeiten, den Griff des trockenen und nassen Haares sowie den Glanz. Unter dem Begriff Silikone versteht der Fachmann mehrere Strukturen siliciumorganischer Verbindungen.

Dimethiconole (S1) bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

(SiOHR¹₂) - O - (SiR²₂ - O -)ₓ - (SiOHR¹₂) (S1 - I)

Verzweigte Dimethiconole können durch die Strukturformel (S1 - II) dargestellt werden:

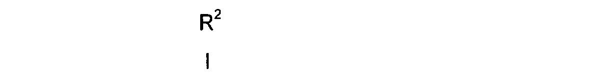

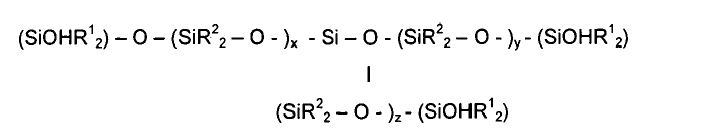

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconole bereits als Emulsion vorliegen können. Dabei kann die entsprechende Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt. Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel Sl 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Wenn die Dimethiconole (S1) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconol bezogen auf die Zusammensetzung.

Erfindungsgemäß besonders geeignet sind auch die sogenannten Dimethicone (S2). Diese können sowohl linear als auch verzweigt als auch cyclisch oder cyclisch und verzweigt sein. Lineare Dimethicone können durch die folgende Strukturformel (S2 - I) dargestellt werden:

(SiR¹₃) - O - (SiR²₂ - O -)ₓ - (SiR¹₃) (S2 - I)

Verzweigte Dimethicone können durch die Strukturformel (S2 - II) dargestellt werden:

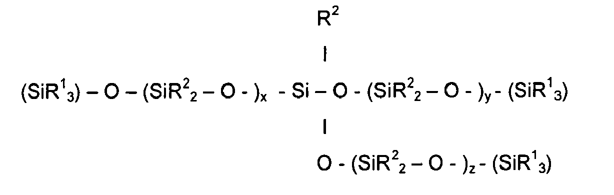

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000.

Insbesondere bevorzugt sind erfindungsgemäß die linearen Polydialkylsiloxane, die Polyalkylarylsiloxane, die Polydiarylsiloxane und/oder die Dihydroxypolydimethylsiloxane.

Die Molgewichte der erfindungsgemäß geeigneten Dimethicone liegen zwischen 1000 D und 10000000 D.

Die Viskositäten der erfindungsgemäß geeigneten Dimethicone liegen üblicherweise zwischen 0,01 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 0,1 und 5000000 cPs, ganz besonders bevorzugte Vsikositäten liegen zwischen 0,1 und 3000000 cPs. Der am meisten bevorzugte Viskositätsbereich der Dimethicone liegt zwischen 0,6 und 600000 cPs.

Selbstverständlich umfasst die erfindungsgemäße Lehre auch, dass die Dimethicone bereits als Emulsion vorliegen können. Dabei kann die entsprechende Emulsion der Dimethicone sowohl nach der Herstellung der entsprechenden Dimethicone aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethicone auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen. Wenn die erfindungsgemäßen Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethicone verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethicone ganz besonders bevorzugt sein.

Wenn die Dimethicone (S2) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethicon, bezogen auf die Zusammensetzung.

Dimethiconcopolyole (S3) bilden eine weitere Gruppe bevorzugter Silikone. Dimethiconole können durch die folgende Strukturformeln dargestellt werden:

(SiR¹₃)-O-(SiR²₂-O-)ₓ-(SiRPE-O-)_{y}-(SiR¹₃) (S3-I)

oder durch die nachfolgende Strukturformel:

PE - (SiR¹₂) - O - (SiR²₂ - O -)ₓ -(SiR¹₂) - PE (S3 - II)

Verzweigte Dimethiconcopolyole können durch die Strukturformel (S3 - III) dargestellt werden: 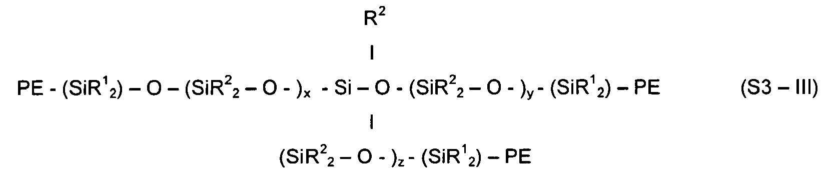 oder durch die Strukturformel(S3 - IV):

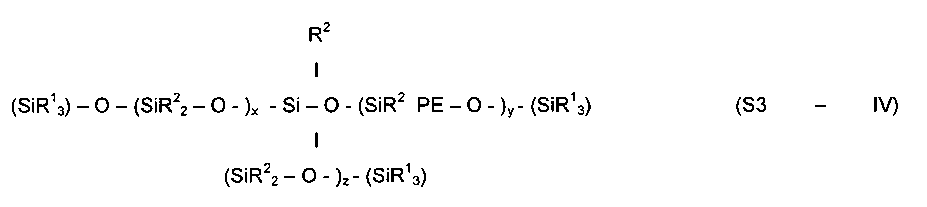

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein. Bevorzugt als R¹ und R² sind Methyl, Phenyl und C2 bis C22 - Alkylreste.

Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconcopolymere bereits als Emulsion vorliegen können. Dabei kann die entsprechende Emulsion der Dimethiconcopolyole sowohl nach der Herstellung der entsprechenden Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconcopolyole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.
Wenn die erfindungsgemäßen Dimethiconcopolyole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconcopolyole ganz besonders bevorzugt sein.

Wenn die Dimethiconcopolyole (S3) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconcopolyol bezogen auf die Zusammensetzung.

Aminofunktionelle Silikone oder auch Amodimethicone (S4) genannt, sind Silicone, welche mindestens eine (gegebenenfalls substituierte) Aminogruppe aufweisen.

Solche Silicone können z.B. durch die Formel (S4 - I)

M(RₐO_{b}SiO_{(4-a-b)/2)x}(R_{c}SiO_{(4-c)/2)y}M (S4 - I)

Beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, - CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)₂(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und z 0 ist.

Q ist am bevorzugtesten ein polarer, aminfunktioneller Rest der Formel - CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (S4 - 11)

R'ₐG₃₋ₐ-Si(OSiG₂)ₙ-(OSiG_{b}R'_{2-b})ₘ-O-SiG₃₋ₐ-R'ₐ (S4 - II),

enthalten, worin bedeutet:
- G ist-H, eine Phenylgruppe, -OH, -O-CH₃, -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -N(R")-CH₂-CH₂- N(R")₂
   o -N(R")₂
   o -N⁺(R")₃A⁻
   o -N⁺H(R")₂ A⁻
   o -N⁺H₂(R")A⁻
   o -N(R")-CH₂-CH₂-N⁺R"H₂A⁻,
   wobei jedes R" für gleiche oder verschiedene Reste aus der Gruppe -H, - Phenyl, -Benzyl, der C₁₋₂₀-Alkylreste, vorzugsweise -CH₃, -CH₂CH₃, - CH₂CH₂CH₃, -CH(CH₃)₂, -CH₂CH₂CH₂H₃, -CH₂CH(CH₃)₂, -CH(CH₃)CH₂CH₃, - C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie ein aminofunktionelles Silikon der Formel (S4 - III) 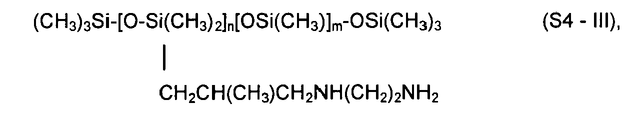 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die dadurch gekennzeichnet sind, daß sie ein aminofunktionelles Silikon der Formel (S4 - IV) 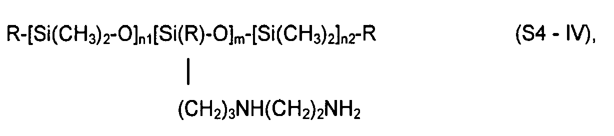 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Wenn die Amodimethicone (S4) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Amodimethicon bezogen auf die Zusammensetzung.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung verwendet. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.%.

Wenn die Silikonmischung in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Silikonmischung bezogen auf die Zusammensetzung.

In einer weiteren bevorzugten Ausführungsform kann die Wirkung der erfindungsgemäßen Wirkstoffkombination durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zu Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüsts eine Hydroxylgruppe tragen und sowohl aus tierischem Gewebe (Zoosterine) wie auch aus pflanzlichen Fetten (Phytosterine) isoliert werden. Beispiele für Zoosterine sind das Cholersterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureestervon Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®}PGPH),
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 - 25 Gew.-%, insbesondere 0,5-15 Gew.-%, bezogen auf das gesamte Mittel.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Als weiterhin vorteilhaft hat es sich gezeigt, dass weitere Polymere die Wirkung der erfindungsgemäßen Zubereitungen weiter unterstützen können. In einer bevorzugten Ausführungsform werden den erfindungsgemäßen Mitteln daher Polymere zugesetzt, wobei sich sowohl anionische, amphotere als auch nichtionische Polymere als wirksam erwiesen haben.

Bei den anionischen Polymeren, welche die Wirkung der erfindungsgemäßen Zubereitungen unterstützen können, handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylenbisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnungg Stabileze^{®} QM im Handel erhältlich.

Weiterhin können als Polymere zur Steigerung der Wirkung der erfindungsgemäßen Zubereitungen amphotere Polymere verwendet werden. Unter dem Begriff amphotere Polymere werden sowohl solche Polymere, die im Molekül sowohl freie Aminogruppen als auch freie - COOH- oder SO₃H-Gruppen enthalten und zur Ausbildung innerer Salze befähigt sind, als auch zwitterionische Polymere, die im Molekül quartäre Ammoniumgruppen und -COO-- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-lon ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren Ausführungsform nichtionogene Polymere enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone gemäß der EP 0612759 B1.

Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten.

Die Polymere sind in den erfindungsgemäßen itteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- Entschäumer wie Silikone,
- Farbstoffe zum Anfärben des Mittels,
- Weitere Antischuppenwirkstoffe wie Piroctone Olamine, Zink Omadine und Climbazol,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Die erfindungsgemäßen Reinigungsmittel unterliegen hinsichtlich ihrer Konfektionierungsform keinerlei Beschränkungen und können als Emulsion, Creme, Lösung, Gel oder Mousse formuliert werden. Bevorzugte Ausführungsformen des erfindungsgemäßen Mittels sind Shampoos, Duschbäder, Duschgele, Haarspülungen, Haarkuren, Haarwässer, Haargele, Haarsprays, Haarschäume, Haarfestiger, Rasierwässer oder Deodorantien.

Ein zweiter Gegenstand der Erfindung ist ein kosmetisches Verfahren zur Stabilisierung von ein- oder mehrschichtig modifizierten, nanopartikulären Metalloxidpartikeln in tensidischen Sytemen, bei dem gehärtetes Rizinusöl in die tensidische Mischung, enthaltend das unlösliche Metalloxid, eingebracht wird.

Besonders bevorzugt ist ein erfindungsgemäßes Verfahren, bei dem ein- oder mehrschichtig modifiziertes, nanopartikuläres Zinkoxid in tensidischen Systemen stabilisiert wird.

Weiterhin besonders bevorzugt ist ein Verfahren, bei dem das gehärtete Rizinusöl über einen Heißprozess feinverteilt oder als feingemahlenes Pulver in die tensidische Mischung eingebracht wird.

Ein dritter Gegenstand der Erfindung ist die kosmetische Verwendung von gehärtetem Rizinusöl zur Stabilisierung von ein- oder mehrschichtig modifizierten, nanopartikulären Metalloxidpartikeln in tensidischen Sytemen.

Ein vierter Gegenstand der Erfidnung ist die kosmetische Verwendung von gehärtetem Rizinusöl zur Stabilisierung von ein- oder mehrschichtig modifizierten, nanopartikulären Zinkoxidpartikeln in tensidischen Sytemen.

Besonders bevorzugt ist die kosmetische Verwendung in einem tensidischen System mit einem pH-Bereich von 4,0 bis 9,0, insbesondere von 7,0 bis 8,0.

Die folgenden Beispiele sollen die Erfindung näher erläutern, ohne sie darauf zu beschränken.

Die aufgeführten Mengenangaben in den Beispielen beziehen sich, sofern nicht anders angegeben, auf Gew.-%.

### Beispiele:

Es wurden die folgenden, erfindungsgemäßen Shampoozubereitungen hergestellt:

| Rohstoff-Gruppe | Rohstoff | Formulierungen | | | |
|---|---|---|---|---|---|
| | | 1 | 2 | 3 | 4 |
| 1 | Wasser | ad 100 | ad 100 | ad 100 | ad 100 |
| 1A | Sodium Laureth Sulfate | 10,00 | 10,00 | | |
| 1A | Sodium Lauryl Sulfate | | | 2,50 | 2,50 |
| 1A | Ammonium Laureth Sulfate | | | 7,50 | 7,50 |
| 1B | Hydrogenated Castor Oil | 0,50 | 0,25 | 0,25 | |
| 1C | Dimethicone 60,000 cSt | 0,60 | 0,60 | | |
| 1C | Dimethicone 300,000 cSt | | | 1,30 | 1,30 |
| 1D | Cetylalkohol | | | 0.30 | 0,30 |
| 1D | Glycol Distearate | 0,80 | 0,80 | | |
| 1D | StyreneNP Copolymer | | | 0,30 | 0,30 |
| 1D | Modifiziertes Zinkoxid | 1,00 | 1,00 | 1,00 | 1,00 |
| 2 | Wasser | 5,00 | 5,00 | 5,00 | 5,00 |
| 2 | Sodium Hydroxide | 0,01-0,50 | 0,01-0,50 | 0,01-0,50 | 0,01-0,50 |
| 3 | Wasser | 35,00 | 35,00 | 35,00 | 35,00 |
| 3A | Polyquaternium-10 | 0,15 | 0,15 | | |
| 3A | Polyquaternium-7 | | | 0,30 | 0,30 |
| 3B | Panthenol | 0.50 | 0,50 | | |
| 3B | Niacinamide | 0,30 | 0,30 | | |
| 3B | Hydrolyzed Wheat Protein | | | 0,20 | 0,20 |
| 3B | Pantolacton | | | 0,30 | 0,30 |
| 3B | Laurdimonium Hydroxypropyl Hydrolyzed Wheat Protein | | | 0,10 | 0,10 |
| 3C | Konservierungsmittel (Sodium Salicylate, Sodium Benzoate, Phenoxyethanol, Methylparabene, Propylparabene und/oder Butylparabene) | 0,10-1,00 | 0,10-1,00 | 0,10-1,00 | 0,10-1,00 |
| 4A | Cocamidopropyl Betaine | | | 3,50 | 3,50 |
| 4A | Disodium Cocoamphodiacetate | 3,00 | 3,00 | | |
| 4B | Laureth-2 | | | 0,30 | 0,30 |
| 4B | PEG-120 Methyl Glucose Dioleate | 0,20 | 0,20 | | |
| 4C | Parfum | 0,50 | 0,50 | 0,50 | 0,50 |
| 5 | Ansäuerungsmittel (Citric Acid und/oder Lactic | 0,10- | 0,10- | 0,10- | 0,10- |
| | Acid) | 0,50 | 0,50 | 0,50 | 0,50 |
| 6 | NaCl | 0,05- | 0,05- | 0,05- | 0,05- |
| | | 1,00 | 1,00 | 1,00 | 1,00 |
| | pH-Wert | 7,0-8,0 | 7,0-8,0 | 7,0-8,0 | 7,0-8,0 |
| | Viskosität (Brookfield DV2 + 20°C / 20UPM) | 7000- | 7000- | 7000- | 7000- |
| | mPas | 9000 | 9000 | 9000 | 9000 |
| Stabilitätstest nach 12 Wochen Lagerung bei 45°C | | k.V. | k.V. | k.V. | S.E. |

| | | | | | |
|---|---|---|---|---|---|
| k.V. : keine Veränderung S.E. : Separationserscheinungen | | | | | |

## Patentansprüche

1. Tensidhaltiges Mittel zur Behandlung keratinischer Fasern, **dadurch gekennzeichnet, dass** es zur Stabilisierung darin unlöslicher Metalloxid-Partikel gehärtetes Rizinusöl enthält.

2. Mittel nach Anspruch 1, **dadurch gekennzeichnet, dass** es ein- oder mehrfach beschichtete, nanopartikuläre Metalloxid-Partikel enthält.

3. Mittel nach einem der Ansprüche 1 oder 2, **dadurch gekennzeichnet, dass** es - bezogen auf das gesamte Mittel - 0,05 bis 10,0 Gew.-%, bevorzugt 0,1 bis 5 Gew.-% und insbesondere 0,5 bis 2,0 Gew.-% ein- oder mehrschichtig modifiziertes, nanopartikuläres Metalloxid enthält.

4. Mittel nach einem der Ansprüche 1 bis 3, **dadurch gekennzeichnet, dass** die Metalloxid-Partikel mit Alkylethercarbonsäuren und Polyetheralkylethern oder Polyethylenglycolalkylethern beschichtet sind.

5. Mittel nach einem der Ansprüche 1 bis 4, **dadurch gekennzeichnet, dass** die Metalloxid-Partikel zusammengesetzt sind aus
- 60 bis 75 Gew.% nanopartikulärem Metalloxid,
- 5 bis 25 Gew.-% Alkylethercarbonsäure(n) und
- 5 bis 25 Gew.-% Polyetheralkylether(n) oder Polyethylenglycolalkylethern.

6. Mittel nach einem der Ansprüche 1 bis 5, **dadurch gekennzeichnet, dass** die Alkylethercarbonsäure(n) eine Alkylkettenlänge von 6 bis 22 C-Atomen, bevorzugt von 8 bis 18 C-Atomen und insbesondere von 10 bis 14 C-Atomen aufweist(en).

7. Mittel nach einem der Ansprüche 1 bis 6, **dadurch gekennzeichnet, dass** der Polyetheralkylether einen Ethoxylierungsgrad von 0 bis 20, insbesondere von 8 bis 12, und eine Alkylkettenlänge von 4 bis 22 C-Atomen, bevorzugt von 6 bis 18 C-Atomen und insbesondere von 8 bis 16 C-Atomen aufweist.

8. Mittel nach einem der Ansprüche 1 bis 7, **dadurch gekennzeichnet, dass** das Metalloxid ausgewählt ist aus Zinkoxid und/oder Titanoxid, insbesondere aus Zinkoxid.

9. Mittel nach einem der Ansprüche 1 bis 8, **dadurch gekennzeichnet, dass** es einen pH-Bereich von 4,0 bis 9,0, insbesondere von 7,0 bis 8,0 aufweist.

10. Mittel nach einem der Ansprüche 1 oder 9, **dadurch gekennzeichnet, dass** es ein Gemisch aus mindestens einem anionischen Tensid mit einem amphoteren und/oder zwitterionischen und/oder einem nichtionischen Tensid umfasst.

11. Mittel nach einem der Ansprüche 1 bis 10, **dadurch gekennzeichnet, dass** es - bezogen auf das gesamte Mittel - 5 bis 35 Gew.-%, bevorzugt 7 bis 25 Gew.-% und insbesondere 8 bis 15 Gew.-% Tenside enthält.

12. Mittel nach einem der Ansprüche 1 bis 11, **dadurch gekennzeichnet, dass** es - bezogen auf das gesamte Mittel - 0,01 bis 5 Gew.-%, bevorzugt 0,05 bis 3 Gew.-% und insbesondere 0,1 bis 2 Gew.-% gehärtetes Rizinusöl enthält.

13. Mittel nach einem der Ansprüche 1 bis 12, **dadurch gekennzeichnet, dass** es - bezogen auf das gesamte Mittel - 0,0005 bis 0,1 Gew.-%, bevorzugt 0,001 bis 0,05 Gew.-% und insbesondere 0,005 bis 0,01 Gew.-% eines kosmetischen Konservierungsmittels enthält.

14. Mittel nach einem der Ansprüche 1 bis 13, **dadurch gekennzeichnet, dass** das Konservierungsmittel ausgewählt ist aus Benzoesäure, Propionsäure, Salicylsäure und/oder Sorbinsäure, sowie aus den kosmetisch verträglichen Salzen und Estern dieser Säuren, aus Formaldehyl und/oder Paraformaldehyd, aus Benzylalkohol, Germall, Dowicil, Kathon, Baypival, Methylisothiazoline, quaternären Ammoniumverbindungen, Alkoholen und/oder aus etherischen Ölen wie Teebaumöl, Cineol, Eugenol, Geraniol, Limonen, Menthol und/oder Thymol.

15. Mittel nach Anspruch 14, **dadurch gekennzeichnet, dass** das Konservierungsmittel aus Benzoesäure, Salicylsäure und/oder einem kosmetisch verträglichen Salz oder Ester dieser Säuren, Benzylalkohol, Dowicil, Kathon, Baypival, Methylisothiazoline sowie aus Gemischen dieser Stoffe, insbesondere aus Methylisothiazoline oder einem Gemisch aus Methylisothiazoline mit einem der Stoffe, ausgewählt ist.

16. Mittel nach einem der Ansprüche 1 bis 15, **dadurch gekennzeichnet, dass** es - bezogen auf das gesamte Mittel - 0,05 bis 5 Gew.-%, bevorzugt 0,1 bis 3,0 Gew.-% und insbesondere 0,3 bis 2,0 Gew.-% eines oder mehrerer Antischuppenwirkstoffe(s) enthält.

17. Mittel nach Anspruch 16, **dadurch gekennzeichnet, dass** der oder die Antischuppenwirkstoff(e) ausgewählt ist (sind) aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

18. Mittel nach einem der Ansprüche 1 bis 17, **dadurch gekennzeichnet, dass** es weiterhin mindestens einen Vertreter aus der Gruppe der zwitterionischen, nichtionischen, kationischen Tenside oder aus Gemischen davon, der kationischen Polymere, der wasserunlöslichen Wachs- und Ölkomponenten, der Vitamine, der Provitamine, der Proteinhydrolysate, der Pflanzenextrakte, der UV-Filter, der Aminosäuren, der wasserunlöslichen Silikone, der wasserlöslichen Silikone und/oder der Amodimethicone enthält.

19. Kosmetisches Mittel nach einem der Ansprüche 1 bis 15, **dadurch gekennzeichnet, dass** es als Shampoo, Duschbad, Duschgel, Haarspülung, Haarkur, Haarwasser, Haargel, Haarspray, Haarschaum, Haarfestiger, Rasierwasser oder Deodorant vorliegt.

20. Verfahren zur Stabilisierung von ein- oder mehrschichtig modifizierten, nanopartikulären Metalloxidpartikeln in tensidischen Sytemen, **dadurch gekennzeichnet, dass** gehärtetes Rizinusöl in die tensidische Mischung, enthaltend das unlösliche Metalloxid, eingebracht wird.

21. Verfahren nach Anspruch 20, **dadurch gekennzeichnet, dass** ein- oder mehrschichtig modifiziertes, nanopartikuläres Zinkoxid in tensidischen Systemen stabilisiert wird.

22. Verfahren nach einem der Ansprüche 20 oder 21, **dadurch gekennzeichnet, dass** das gehärtete Rizinusöl über einen Heißprozess feinverteilt oder als feingemahlenes Pulver in die tensidische Mischung eingebracht wird.

23. Kosmetische Verwendung von gehärtetem Rizinusöl zur Stabilisierung von ein- oder mehrschichtig modifizierten, nanopartikulären Metalloxidpartikeln in tensidischen Sytemen.

24. Kosmetische Verwendung von gehärtetem Rizinusöl zur Stabilisierung von ein- oder mehrschichtig modifizierten, nanopartikulären Zinkoxidpartikeln in tensidischen Sytemen.

25. Kosmetische Verwendung nach einem der Ansprüche 23 oder 24 in einem tensidischen System mit einem pH-Bereich von 4,0 bis 9,0, insbesondere von 7,0 bis 8,0.
